# EUROPEAN PATENT APPLICATION

(11) **EP 4 306 545 A1**
(43) Date of publication of application: **17.01.2024**
(21) Application number: 22766405.9
(22) Date of filing: 11.03.2022
(51) Int. Cl.: C07K 19/00, C12N 15/62, A61K 38/16, A61P 37/02, A61P 35/00

(54) **FUSION PROTEIN OF TNFR2 AND APRIL BAFF RECEPTOR**

(30) Priority: 12.03.2021 CN 202110272529
(71) Applicant: Shanghai Celgen Bio-Pharmaceutical Co., Ltd., Shanghai 201203 (CN)
(72) Inventor: CAI, Zeling, Shanghai 201203 (CN); CHEN, Yi, Shanghai 201203 (CN); LI, Peng, Shanghai 201203 (CN)
(74) Representative: Santoro, Sofia
(86) International application number: PCT/CN2022/080467
(87) International publication number: WO 2022/188883

(57) **Abstract**

The present invention provides a preparation method and use of a TNFR2 and APRIL/BAFF receptor protein fusion. Specifically, the present invention provides a fusion protein, the fusion protein comprising a TNFR2 extracellular segment and a BAFFR or BCMA or TACI extracellular segment. The fusion protein of the present invention inhibits TNF α-induced inflammation, and also by means of combining with BAFF or APRIL, inhibits the activation of B cells, and plays a synergistic role with TNFR2, thus enhancing the effect of treating various autoimmune diseases.

## Description

### Technical field

The present invention relates to the field of biotechnology. Specifically, it relates to a fusion protein of TNFR2 and APRIL/BAFF receptor.

### Background

Autoimmune disease is a kind of disease in which autocells and humors react to their own body. At present, the incidence rate and prevalence rate of autoimmune diseases worldwide are increasing year by year. Common autoimmune diseases include rheumatoid arthritis (RA), ankylosing spondylitis (AS), psoriasis (PS), systemic lupus erythematosus (SLE), etc., which have a serious impact on patients' work ability and quality of life. In recent years, TNF α targeted inhibitors which effectively control the progression of diseases by antagonizing TNF α and inhibiting the upregulation of its downstream cytokines IL-2, IL-6, IFN- γ, etc., while inhibiting overactivated T cells, are widely used in various autoimmune diseases. Although TNF α inhibitors have achieved good therapeutic effects, but there are still 50% of patients whose treatment effects are not ideal. Therefore, there is an urgent need for new combinations to achieve more effective control of autoimmune diseases.

The present invention provides a safer, more effective, and more precise therapeutic fusion protein for autoimmune diseases.

### Summary of the invention

The purpose of the present invention is to provide a safer, more effective, and more precise therapeutic fusion protein for autoimmune diseases.

In a first aspect of the present invention, it provides a fusion protein, wherein the fusion protein comprises the the following fused elements:
(a) TNF receptor or active fragment thereof;
(b) APRIL/BAFF receptor or active fragment thereof, wherein the APRIL/BAFF receptor includes TACI, BCMA, BAFFR, and combinations thereof;
   and optionally (c) antibody Fc region;
   wherein the fusion protein retains the biological activity of above elements (a) and (b).

In another preferred embodiment, the TNF receptor is selected from the following group consisting of: TNFR2, TNFR1, and combinations thereof.

In another preferred embodiment, the TNF receptor is derived from a human or non-human mammal, preferably from a rodent (such as mouse, rat), primate and human.

In another preferred embodiment, the TNF receptor comprises a wild-type or a mutant-type.

In another preferred embodiment, the TNF receptor comprises a full-length, mature form of the TNF receptor, or an active fragment thereof.

In another preferred embodiment, the TNF receptor further comprises a TNF receptor derivative.

In another preferred embodiment, the TNF receptor derivative comprises a modified TNF receptor, a protein molecule with amino acid sequence homologous to natural TNF receptors and activity of natural TNF receptors, a dimer or polymer of the TNF receptor, and a fusion protein containing the amino acid sequence of the TNF receptor.

In another preferred embodiment, the modified TNF receptor is a pegylated TNF receptor.

In another preferred embodiment, the "protein molecule with amino acid sequence homologous to natural TNF receptors and activity of natural TNF receptors" refers to a protein molecule whose amino acid sequence has ≥ 85% homology, preferably ≥ 90% homology, more preferably ≥ 95% homology, and most preferably≥ 98% homology with the TNF receptors; and with TNF receptor activity.

In another preferred embodiment, the TNF receptor comprises a first domain, a second domain, a third domain, and/or a fourth domain.

In another preferred embodiment, the first domain, second domain, third domain, and/or fourth domain are each independently cysteine-rich domains (CRDs).

In another preferred embodiment, the TNF receptor contains or has residues 1-235, 17-179, 17-140, 55-179, or 55-140 of the TNFR2 amino acid sequence (SEQ ID NO.:13).

In another preferred embodiment, the APRIL/BAFF receptor is derived from a human or non-human mammal, preferably from a rodent (such as mouse, rat), primate and human.

In another preferred embodiment, the APRIL/BAFF receptor comprises a wild-type or a mutant-type .

In another preferred embodiment, the APRIL/BAFF receptor further comprises an APRIL/BAFF receptor derivative.

In another preferred embodiment, the APRIL/BAFF receptor derivative comprises a modified APRIL/BAFF receptor, a protein molecule with amino acid sequence homologous to natural APRIL/BAFF receptors and activity of natural APRIL/BAFF receptors, a dimer or polymer of the APRIL/BAFF receptor, and a fusion protein containing the amino acid sequence of the APRIL/BAFF receptor.

In another preferred embodiment, the "protein molecule with amino acid sequence homologous to natural TNF receptors and activity of natural TNF receptors" refers to a protein molecule whose amino acid sequence has ≥ 85% homology, preferably ≥ 90% homology, more preferably ≥ 95% homology, and most preferably≥ 98% homology with the TNF receptors; and with APRIL/BAFF receptor activity.

In another preferred embodiment, the APRIL/BAFF receptor is selected from the following group consisting of: TACI, BCMA, BAFFR, and combinations thereof.

In another preferred embodiment, the TACI contains or has residues 1-109, 33-109, or 68-109 of the TACI amino acid sequence (SEQ ID NO.:14).

In another preferred embodiment, the BCMA contains or has residues 1-54, or 6-54 of the BCMA amino acid sequence (SEQ ID NO.:15).

In another preferred embodiment, the BAFFR contains or has residues 1-78, 12-78, or 12-46 of the BAFFR amino acid sequence (SEQ ID NO.: 16).

In another preferred embodiment, the Fc region is derived from a human or non-human mammal, preferably from a rodent (such as mouse, rat), primate and human.

In another preferred embodiment, the Fc fragment is the Fc fragment of immunoglobulin IgG, preferably the Fc part of IgG1.

In another preferred embodiment, the Fc fragment comprises a native Fc fragment and an Fc mutant.

In another preferred embodiment, the Fc fragment contains or has residues 99-329 of the human IgG1 amino acid sequence (accession number UniProtKB-P01857).

In another preferred embodiment, the amino acid sequence of the Fc fragment is as shown in SEQ ID NO.:17.

In another preferred embodiment, the fusion protein also has one or more of the following functions:
(a) activity of binding TNFα;
(b) inhibition of TNFα-induced inflammation;
(c) activity of binding BAFF or APRIL;
(d) inhibiting or blocking the BAFF/APPRIL pathway;
(e) reducing the number of B cells *in vivo*;
(f) reducing the content of B lymphocytes in the spleen or blood;
(g) inhibiting splenic enlargement;
(h) inhibition of APRIL/BAFF-induced diseases.

In another preferred embodiment, the fusion protein has the following dimeric structure as shown in formula I or II:

X-Y-Z (I)

Y-X-Z (II);

wherein,
X is an extracellular segment of the TNF receptor;
Y is an extracellular segment of TACI, BCMA or BAFFR;
Z is none or an optional Fc region of human antibody;
and "-" is a peptide bond or peptide linker.

In another preferred embodiment, the fusion protein is a homodimer.

In another preferred embodiment, the amino acid sequence of the fusion protein is as shown in SEQ ID NO.: 18 or 19 or 20.

In another preferred embodiment, any two of the X, Y and Z are connected in a head to head, head to tail, or tail to tail manner.

In another preferred example, the "head" refers to the N-terminus of a peptide or fragment thereof, especially the N-terminus of a wild-type peptide or fragment thereof.

In another preferred embodiment, the "tail" refers to the C- terminus of a peptide or fragment thereof, especially the C-terminus of a wild-type peptide or fragment thereof.

In another preferred embodiment, the peptide linker is a peptide linker with a length of 1-20 amino acids, preferably 1-10 amino acids.

In another preferred embodiment, the X contains or has residues 1-235, 17-179, 17-140, 55-179, or 55-140 of the TNFR2 amino acid sequence (SEQ ID NO.:13); and/or
the Y contains or has:
(a) residues 1-109, 33-109, or 68-109 of the TACI amino acid sequence (SEQ ID NO.:14);
(b) residues 1-54, or 6-54 of the BCMA amino acid sequence (SEQ ID NO.:15); or
(c) residues 1-78, 12-78, or 12-46 of the BAFFR amino acid sequence (SEQ ID NO.:16).

In another preferred embodiment, the Z contains or has residues 99-329 of the human IgG1 amino acid sequence (accession number UniProtKB-P01857).

In a second aspect of the present invention, it provides a nucleic acid moleculeencoding the fusion protein of the first aspect of the present invention.

In another preferred embodiment, the nucleic acid molecule also contains additional auxiliary elements selected from the following group on the side of the ORF of the mutant or fusion protein: signal peptides, secreted peptides, tag sequences (such as 6His), and combinations thereof.

In another preferred embodiment, the nucleic acid molecule is selected from the following group: DNA sequences, RNA sequences, and combinations thereof.

In a third aspect of the present invention, it provides a vector comprising the nucleic acid molecule of the second aspect of the present invention.

In another preferred embodiment, the vector comprises one or more promoters operatively connected to the nucleic acid sequence, enhancer, transcription termination signal, polyadenylation sequence, replication origin, selective labeling, nucleic acid restriction site, and/or homologous recombination site.

In another preferred embodiment, the vector comprises a plasmid or a viral vector.

In another preferred embodiment, the viral vector is selected from the group consisting of an adeno-associated virus (AAV), adenovirus, lentivirus, retrovirus, herpesvirus, SV40, poxvirus, and combinations thereof.

In another preferred embodiment, the vector comprises an expression vector, a shuttle vector and an integration vector.

In a fourth aspect of the present invention, it provides a genetically engineered cell which comprises the vector of the third aspect of the present invention; or has the nucleic acid molecule of the second aspect of the present invention integrated into its genome.

In another preferred embodiment, the genetically engineered cell is a eukaryotic cell, such as a yeast cell, plant cell, or mammalian cell (including human and non-human mammals).

In another preferred embodiment, the genetically engineered cell is a prokaryotic cell, such as *Escherichia coli.*

In another preferred embodiment, the genetically engineered cell is selected from the following group: *Escherichia coli*, wheat germ cell, insect cell, SF9, SP2/0, Hela, HEK293, CHO (such as CHOKS), yeast cell, and combinations thereof.

In a fifth aspect of the present invention, it provides a method for producing the fusion protein of the first aspect of the present invention, which comprises the steps of:
culturing the genetically engineered cell of the fourth aspect of the present invention under conditions suitable for expression, thereby expressing the fusion protein; and
isolating or purifying the fusion protein.

In a sixth aspect of the present invention, it provides a pharmaceutical composition, comprising the fusion protein of the first aspect of the present invention and a pharmaceutically acceptable carrier thereof.

In another preferred embodiment, the pharmaceutical composition further comprises other drugs for treating immune diseases.

In another preferred embodiment, other drugs for treating immune diseases are selected from the following group: hormone drugs, immunosuppressive drugs, small molecule targeted drugs, biological agents, and combinations thereof.

In another preferred embodiment, the hormone drugs are selected from the following group: cortisone, hydrocortisone, prednisone, prednison, prednisolone, dexamethasone, betamethasone, and combinations thereof.

In another preferred embodiment, the immunosuppressive drugs are selected from the following group: methotrexate, cyclophosphamide, imidazolium thioprine, cyclosporine, mycophenolate mofetil, tacrolimus, sirolimus, leflunomide, sulfamethoxazole, hydroxychloroquine, and combinations thereof.

In another preferred embodiment, the small molecule targeted drugs are selected from the following group: tofacitinib, baricitinib, and combinations thereof.

In another preferred embodiment, the biological agents are selected from the following group: etanercept, sacituzumab, adalimumab, golimumab, infliximab, tocilizumab, secukinumab, ustekinumab, canakinumab, anakinra, rilonacept, abatacept, rituximab, belimumab, and combinations thereof.

In a seventh aspect of the present invention, it provides a use of the fusion protein of the first aspect of the present invention, the nucleic acid molecule of the second aspect of the present invention, the vector of the third aspect of the present invention, or the genetically engineered cell of the fourth aspect of the present invention, for the preparation of one or more compositions selected from the following group:
(a) composition for inhibiting BAFF/APPRIL signaling pathway;
(b) composition for treating immune diseases; and/or
(c) composition for treating B cell proliferation-related diseases.

In another preferred embodiment, the composition is a pharmaceutical composition.

In another preferred embodiment, the immune diseasesare selected from the following group: rheumatoid arthritis (RA), ankylosing spondylitis (AS), psoriasis (PS), psoriatic arthritis (PsA), juvenile idiopathic arthritis (JIA), systemic lupus erythematosus (SLE), Behcet's disease (BD), multiple sclerosis (MS), Sjogren's syndrome (SS), Graves disease, Crohn's disease (CD), ulcerative colitis (UC), primary glomerulonephritis, IgA nephropathy, autoimmune vasculitis, polymyositis (PM), non-infectious uveitis, autoimmune hemolytic anemia (AIHA), autoimmune purpura (ATTP), N-methyl-d-aspartate receptor (NMDAR) encephalitis, myasthenia gravis, hydradenitis suppurativa (HS), myelin oligodendrocyte glycoprotein spectrum disorder (MOGSD), and neuromyelitis optica spectrum disorder (NMOSD), and combinations thereof.

In another preferred embodiment, the immune diseases include autoimmune diseases such as rheumatoid arthritis (RA), ankylosing spondylitis (AS), psoriasis (PS), psoriatic arthritis (PsA), juvenile idiopathic arthritis (JIA), systemic lupus erythematosus (SLE), autoimmune purpura (ATTP), Behcet's disease, and IgA nephropathy.

In another preferred embodiment, the B cell proliferation-related diseases include multiple myeloma, chronic lymphocytic leukemia, megaglobulinemia, and plasma cell leukemia.

In an eighth aspect of the present invention, it provides a method of treating an immune disease by administering the fusion protein of the first aspect of the present invention to a patient in need thereof.

In another preferred embodiment, the fusion protein is administered in a monomeric and/or dimeric form.

In another preferred embodiment, the immunological diseases comprise rheumatoid arthritis (RA), ankylosing spondylitis (AS), psoriasis (PS), psoriatic arthritis (PsA), juvenile idiopathic arthritis (JIA), systemic lupus erythematosus (SLE), Behcet's disease (BD), multiple sclerosis (MS), Sjogren's syndrome (SS), Graves disease, Crohn's disease (CD), ulcerative colitis (UC), primary glomerulonephritis, autoimmune vasculitis, polymyositis (PM), non-infectious uveitis, autoimmune hemolytic anemia (AIHA), autoimmune purpura (ATTP), N-methyl-d-aspartate receptor (NMDAR) encephalitis, myasthenia gravis, hydradenitis suppurativa (HS), myelin oligodendrocyte glycoprotein spectrum disorder (MOGSD), and neuromyelitis optica spectrum disorder (NMOSD).

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Description of the figures

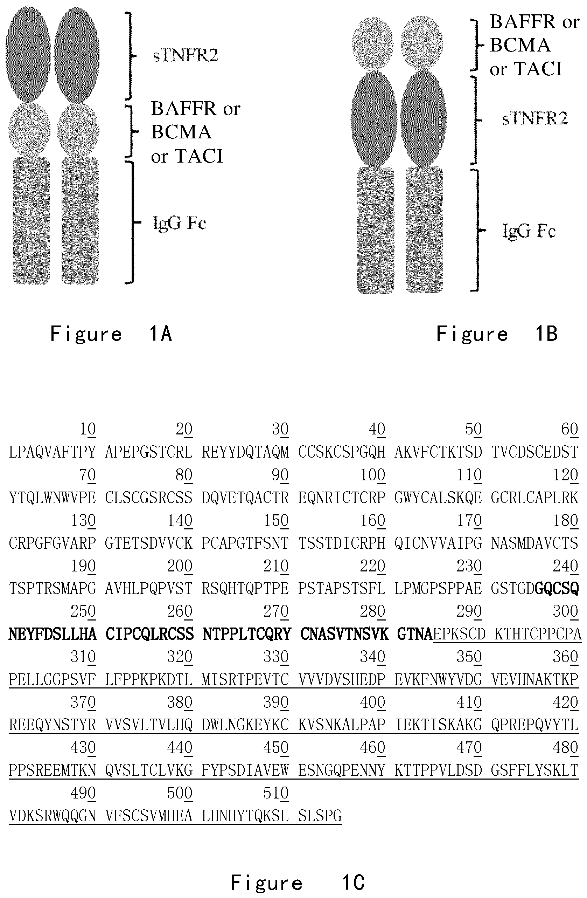

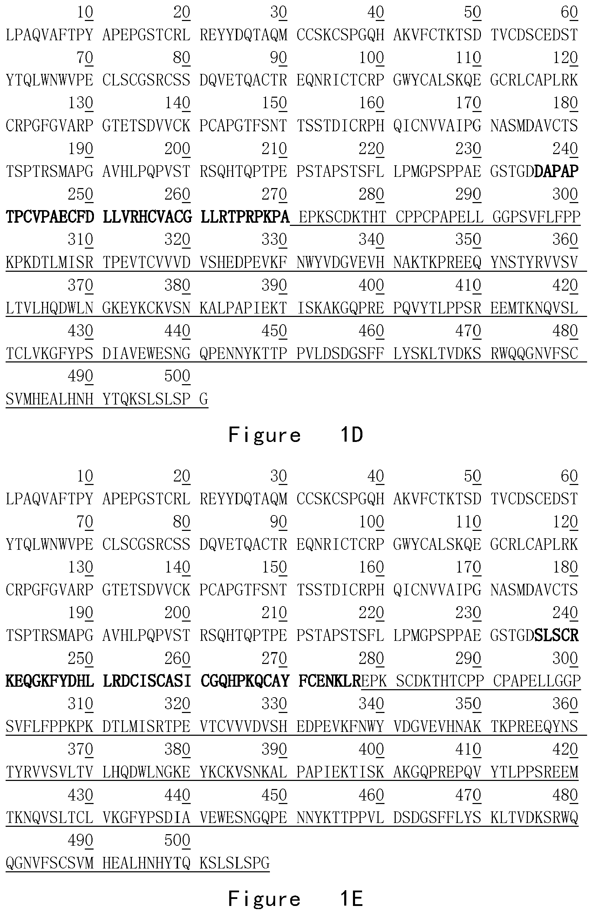

Figure 1 shows a schematic representation of the fusion protein structure (1A and 1B), and Figure 1C shows the amino acid sequence of the TNFR2-BCMA-Fc fusion protein (SEQ ID NO.: 19), wherein the residues 1-235 are a TNFR2 active fragment, the bold-marked part is a BCMA active fragment (residues 236-284) and the underlined part is an IgG Fc fragment (residues 285-515); Figure 1D shows the amino acid sequence of the TNFR2-BAFFR-Fc fusion protein (SEQ ID NO.: 20), wherein the residues 1-235 are a TNFR2 active fragment, the bold-marked part is a BAFFR active fragment (residues 236-270), and the underlined part is an IgG Fc fragment (residues 271-501); Figure 1E shows the amino acid sequence of the TNFR2-TACI-Fc fusion protein (SEQ ID NO.: 18), wherein the residues 1-235 are a TNFR2 active fragment, the bold-marked part is a TACI active fragment (residues 236-277) and the underlined part is an IgG Fc fragment (residues 278-508).
Figure 2 shows the reduced and non-reduced SDS-PAGE electrophoresis analysis results of the fusion proteins (TNFR2-BAFFR-Fc, TNFR2-BCMA-Fc, and TNFR2-TACI-Fc).
Figure 3 shows the *in vitro* binding activity of (3A) TNFR2-BAFFR-Fc fusion proteinor (3B) TNFR2-BCMA-Fc fusion protein to human TNFα.
Figure 4 shows the in *vitro* binding activity of (4A) TNFR2-BCMA-Fc fusion protein or (4B) TNFR2-TACI-Fc fusion protein to human BAFF.
Figure 5 shows the *in vitro* binding activity of (5A) TNFR2-BCMA-Fc fusion protein or (5B) TNFR2-TACI-Fc fusion protein to APRIL.
Figure 6 shows that the *in vitro* binding of TNFR2-BCMA-Fc to TNFa is not affected by BAFF (6A) or APRIL (6B).
Figure 7 shows the activity of TNFR2-BCMA-Fc (7A), or TNFR2-TACI-Fc (7B) fusion protein in inhibiting TNFα-induced apoptosis of L929 cells.
Figure 8 shows the function of TNFR2-BCMA-Fc fusion protein in inhibiting the protection of BAFF (Figure 8A) or APRIL (8B) on RPMI8226 cells from DEX killing, wherein the "control" means the control group.
Figure 9 shows the function of TNFR2-BCMA-Fc fusion protein in inhibiting LPS-induced septic shock death in mice.
Figure 10 shows that TNFR2-BCMA-Fc significantly reduced the number of B cells in the blood (10A) and spleen (10B) of mice, wherein the "etanercept" means the etanercept group.
Figure 11 shows the inhibitory function of TNFR2-BCMA-Fc on inflammation in CIA mice. (11A) shows the dose-effect relationship of TNFR2-BCMA-Fc in inhibiting the degree of inflammation in CIA mice; (11B) shows a comparison of the anti-inflammatory effects between TNFR2-BCMA-Fc and etanercept (Enbrel) at a dose of 5 mg/kg; (11C) shows a comparison of the anti-inflammatory effects between TNFR2-BCMA-Fc and Enbrel at a dose of 20 mg/kg.
Figure 12 shows the results of the toe tissue pathology study on the inhibitory effect of TNFR2-BCMA-Fc on inflammation in CIA mice. (12A) shows the inhibitory effect of TNFR2-BCMA-Fc on synovitis of joint tissue; (12B) shows the inhibitory effect of TNFR2-BCMA-Fc on pannus of joint tissue; (12C) shows the inhibitory effect of TNFR2-BCMA-Fc on bone erosion in joint tissue.
Figure 13 shows the effect of TNFR2-BCMA-Fc on the spleen of CIA mice. (13A) shows the function of TNFR2-BCMA-Fc in reducing the percentage of B lymphocytes in spleen; (13B) shows the function of TNFR2-BCMA-Fc in inhibiting spleen enlargement caused by inflammation.
Figure 14 shows the inhibitory effect of TNFR2-BCMA-Fc on the production of anti-collagen antibodies in CIA mice.

### DETAILED DESCRIPTION

After extensive and intensive research, the inventor accidentally found for the first time that the fusion protein obtained by fusing (a) TNF receptor or its active fragment, (b) TACI, BCMA, BAFFR or its active fragment, and (c) the Fc region of the antibody has excellent biological activity. While inhibiting inflammation induced by TNFα, the fusion protein of the present invention can efficiently recognize key molecules in the immune response (such as BAFF or APRIL), inhibit the activation of B cells, and play a synergistic effect with TNFR2, which helps to treat certain autoimmune diseases. Specifically, the TNFR2-BCMA-Fc fusion protein in the present invention can significantly reduce the content of B lymphocytes in the blood and spleen of mice, inhibit inflammation in CIA mice, and significantly enhance the function of inhibiting spleen enlargement compared to etanercept. On this basis, the present invention has been completed.

Specifically, the inventor prepared optimized fusion proteins TNFR2-BAFFR-Fc, TNFR2-BCMA-Fc, and TNFR2-TACI-Fc. Studies have shown that the fusion protein has strong biological activity and can block the binding of BAFF or APRIL to its cell membrane receptor. In addition, the fusion protein of the invention can significantly reduce the content of B lymphocytes in the spleen of C57 mice, inhibit TNFa-induced apoptosis, protect mice from LPS-induced shock death, and alleviate osteoarticular inflammation in rat models of CIA and AIA.

The fusion protein constructed by the present invention combines APRIL/BAFF related receptors with TNFR2, which has the advantages of precise recognition, synergistic effect, and controllable toxicity. While inhibiting TNFα and its downstream pathways to cause autoimmune responses, the binding of BAFF-R, BCMA, TACI to ligands BAFF and APRIL on B cells is blocked by tricging the receptor. The fusion protein will further improve the therapeutic effect on autoimmune diseases by effectively regulating B cells, T cells, and a series of cytokines.

As used herein, unless otherwise specified, Fc refers to the Fc fragment of human immunoglobulin. The term "immunoglobulin Fc region" refers to the constant region of the immunoglobulin chain, especially the carboxyl end or a part of the constant region of the immunoglobulin heavy chain. For example, the immunoglobulin Fc region may include a combination of two or more domains of heavy chains CH1, CH2, and CH3 and the immunoglobulin hinge region. In a preferred embodiment, the Fc region of the immunoglobulin used includes at least one immunoglobulin hinge region, one CH2 domain, and one CH3 domain, preferably missing the CH1 domain.

There are various categories of human immunoglobulins known, such as IgA, IgD, IgE, IgM, and IgG (including four subclasses of IgG1, IgG2, IgG3, and IgG4). Selecting a specific immunoglobulin Fc region from a specific immunoglobulin category and subclass is within the scope of expertise of those skilled in the art. In a preferred example, the immunoglobulin Fc region may choose a coding sequence containing the human immunoglobulin IgG4 subclass Fc region, wherein one immunoglobulin heavy chain 1 domain (CH1) is deleted, but it includes the coding sequences for the hinge region and two domains, CH2, CH3.

As used herein, "comprise", "have" or "include" includes "contains", "mainly consisting of", "essentially consisting of", and "consisting of". The terms "mainly consisting of", "essentially consisting of" and "consisting of" are subordinate concepts of "comprise", "have" or "include".

### A Proliferation Inducing Ligand (APRIL)

APRIL is a ligand of the TNF superfamily, which is an intercellular hormone synthesized within cells and secreted into extracellular. APRIL exists in the form of a homotrimer and can also form a biologically active heterotrimer with BAFF. APRIL mainly exerts physiological functions by binding to receptors BCMA and TACI. APRIL enhances the proliferation and survival of plasma cells, as well as T cell dependent humoral immunity.

### B lymphocyte stimulator factor (BAFF)

BAFF is a ligand of the TNF superfamily, and its structure is a type II membrane binding protein. BAFF is an essential factor for B cell survival and maturation. BAFF exerts physiological effects mainly by binding to its receptors BAFF-R, BCMA and TACI. In the lupus mouse model, a significant positive correlation was found between the content of soluble BAFF protein in serum and the severity of the disease, mainly related to an increase in peripheral blood autoreactive B cells, abnormal activation of B cells, and the production of autoantibodies. In addition, research has found that transgenic mice overexpressed with BAFF are prone to spontaneous SLE.

BAFF-R (BAFF receptor) is a receptor of BAFF that can specifically bind to BAFF. Its structure is a type III transmembrane protein. BAFF-R plays a crucial role in the maturation stage of B cells, promoting B cell proliferation and differentiation into plasma cells by binding to soluble BAFF. It is an important receptor in the early stages of B cell development. After BAFF binding with BAFF-R, a series of downstream pathways of protein synthesis and energy metabolism are activated, thereby prolonging the half-life of immature B cells, transitional B cells, and mature B cells. After BAFF binding with BAFF-R, it can also continuously induce B cell activation though NF-κB pathway and PI3K pathway. Research has shown that there is also an abnormal increase in BAFF expression in patients with systemic lupus erythematosus, and administration of anti-BAFF antibodies can effectively inhibit the binding of soluble BAFF to its three receptors (BAFF-R, BCMA, TACI) in the body, thereby inhibiting the differentiation and survival of B cells, achieving therapeutic effects.

### B cell maturation antigen (BCMA)

BCMA is a member of the tumor necrosis factor receptor superfamily. BCMA and its ligands play an important role in regulating the immune system, especially in regulating proliferation, differentiation, and apoptosis of B cells. BCMA is mainly expressed on mature B cells and plays an important role in maintaining the homeostasis, tolerance, and terminal differentiation of B cells. Its main function is to mediate the long-term survival of plasma cells and maintain long-term humoral immunity. BCMA is known to be closely related to the occurrence and development of multiple myeloma and autoimmune diseases. A proliferation-inducing ligand (APRIL, also known as TALL-2, TRDL-1, and TNFSF-13) is a member of the TNF ligand superfamily, directly related to the development of B lymphocytes, T cell activation, and humoral immunity.

Previous studies have shown that the regulatory effect of BCMA expressed on B cells is closely related to its ligands BAFF and APRIL. APRIL/BAFF combined with BCMA has been proven to be a costimulatory molecule for B cell activation and antibody production both *in vivo* and *in vitro.* Intervention to block BAFF and APRIL in lupus mice significantly reduced the number of B cells and plasma cells in peripheral blood. Research has shown that the binding of BCMA with its ligand, BAFF or APRIL, can initiate the transcription of anti-apoptotic genes in plasma cells, prolonging cell survival time. A study showed that a significant increase in APRIL was found in patients with rheumatoid arthritis, exacerbating disease progression. After intervention with BCMA-Fc fusion protein, it was found that BCMA-Fc fusion protein could act as a deceive receptor to bind to APRIL with high affinity in the body, thereby inhibiting the biological activity of APRIL and controlling the progression of autoimmune diseases.

### Transmembrane activator and calmodulin ligand interactor (TACI)

TACI is the third receptor of BAFF, in addition to BAFF-R and BCMA. TACI is thought to negatively regulate B cell maturation. In addition to binding to BAFF, TACI, like BCMA, can also bind to APRIL with high affinity. BCMA/TACI binding to APRIL or BAFF induces the activation of NF-κB, and activates multiple downstream signaling pathways, leading to an overactivated immune response and ultimately leading to autoimmune diseases. The TACI fusion protein has been proven to bind to free BAFF and APRIL with high affinity both *in vivo* and *in vitro,* and competitively inhibit its binding to receptors on lymphocytes (TACI, BCMA, BAFF-R), thereby reducing the amount of mature B cells and immunoglobulins in the circulation.

### Fusion protein

As used herein, "the fusion protein of the present invention" or "polypeptide" refers to the fusion protein described in the first aspect of the present invention.

As used herein, unless otherwise specified, the fusion protein is an isolated protein that is not associated with other proteins, polypeptides, or molecules, and is a purified product of recombinant host cell culture or as a purified extract.

The present invention provides a fusion protein comprising the following elements: (a) a TNF receptor or an active fragment thereof, (b) APRIL/BAFF receptors such as TACI, BCMA, and BAFFR or an active fragment thereof, and (c) an antibody Fc region. The fusion protein of the present invention may or may not contain a connecting sequence between the various elements (such as between element a and element b, or element b and element c). The connecting sequence is usually a sequence that does not have an impact on two proteins.

The fusion protein of the present invention not only has a longer *in vivo* half-life, but also can more effectively inhibit the concentration of immune disease related antibodies (especially IgE) in the serum.

According to the amino acid sequence provided by the present invention, those skilled in the art can conveniently prepare the fusion protein of the present invention using various known methods. These methods include, but are not limited to, recombinant DNA methods, artificial synthesis, etc.

After learning the amino acid sequence of the fusion protein of the present invention, those skilled in the art can conveniently obtain the gene sequence encoding the fusion protein of the present invention based on the amino acid sequence.

As a preferred method of the present invention, it is particularly suitable for overexpressing the fusion protein of the present invention in eukaryotic cells (preferably CHO cells), comprising the full length (i.e., 1-515 positions) of the TNFR2-BCMA-Fc fusion protein with its amino acid sequence as shown in SEQ ID NO.: 19 or its active fragment, such as the polypeptide (fusion protein) shown in 55-515 positions.

In a preferred embodiment, the fusion protein of the present invention comprises the following elements:
(a) TNF receptor or its active fragment; (b) APRIL/BAFF receptor or its active fragment, wherein the APRIL/BAFF receptor (APRIL and/or BAFF receptor) includes TACI, BCMA, BAFFR or a combination thereof; and optional (c) an antibody Fc region. The fusion protein of the present invention may or may not contain a connecting sequence between the various elements (such as between element a and element b, or element b and element c). The connecting sequence is usually a sequence that does not have an impact on two proteins.

As used herein, the term "fusion protein" also includes variants of fusion proteins with the aforementioned activity, such as TNFR2 TACI-Fc (amino acid sequence as shown in SEQ ID NO.: 18), TNFR2 BCMA-Fc (amino acid sequence as shown in SEQ ID NO.: 19), and TNFR2 BAFFR-Fc (amino acid sequence as shown in SEQ ID NO.: 20). These variants include (but are not limited to): deletion, insertion and/or substitution of 1-3 (usually 1-2, preferably 1) amino acids, and addition or deletion of one or more (usually less than 3, preferably less than 2, preferably less than 1) amino acids at the C- and/or N-terminal. For example, the protein's functions are usually unchanged when an amino acids is substituted by a similar or analogous one. For another example, addition of one or more amino acids to the C-terminal and/or N-terminal usually does not alter the function of the protein. In addition, the terms also include the monomer and polymer of the polypeptide of the present invention. This term also includes linear and nonlinear polypeptides (such as cyclic peptides).

The present invention further includes the active fragments, derivatives and analogs of the fusion protein of the present invention. As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably the conservative amino acid residues) being substituted, or (ii) a polypeptide having substituted group(s) in one or more amino acid residues, or (iii) a polypeptide formed by fusion of the polypeptide of the present invention with another compound (such as the compound that prolongs the half life of the polypeptide, such as polyethylene glycol), or (iv) a polypeptide formed by the fusion of an additional amino acid sequence to the polypeptide sequence (a fusion protein formed by fusion with a leader sequence, a secreted sequence, or a tag sequence such as 6×His). According to the teaching here, these fragments, derivatives and analogs are within the scope commonly known by the skilled person.

A class of preferred active derivatives is the polypeptides formed by replacing at most 3, preferably at most 2, more preferably at most 1 amino acid with the amino acid having similar or analogous property, compared to the amino acid sequence of Formula I or II. These conservatively variant polypeptides are preferably produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides the analogs of the fusion protein of the present invention. These analogs differ from the polypeptides shown in SEQ ID NO.: 18, 19, or SEQ ID NO.:20 either in the amino acid sequence, or in the form of modifications that do not affect the sequence, or in both. Also included are analogs which have residues other than those naturally occurring L-amino acids (e.g., D-amino acids) or non-naturally occurring or synthetic amino acids (e.g., beta- or gamma-amino acids). It should be understood that, the polypeptides of the present invention is not limited to the representative polypeptides listed hereinabove.

Modification (usually do not change the primary structure) include *in vivo* or *in vitro* chemical derivation of polypeptides, e.g., acelylation or carboxylation. Also included is modification of glycosylation, e.g., the polypeptides made by subjecting to the glycosylation modification during its synthesis and processing or in the further processing steps. These modifications can be accompanied by exposing the polypeptide to enzymes which catalyze glycosylation (e.g., mammalian glycosylating or deglycosylating enzymes). Also included are sequences that have phosphorylated amino acid residues, e.g., phosphotyrosine, phosphoserine, phosphothronine, as well as sequences that have been modified to improve their resistance to proteolytic degradation or to optimize solubility properties.

### Expression vector and host cells, preparation method

The present invention also provides an expression vector comprising a sequence encoding the fusion protein of the present invention and an expression regulatory sequence operably connected to it. The term "operably connected" or "operationally connected" refers to a situation where certain parts of a linear DNA sequence can regulate or control the activity of other parts of the same linear DNA sequence. For example, if the promoter controls the transcription of a sequence, it is operably connected to the coding sequence.

The expression and cloning vector may contain one or more screening genes, also known as screening markers. Typical screening genes encoding proteins can (a) resist antibiotics, etc; (b) compensate for nutritional deficiencies or (c) provide critical nutrients that are not present in the culture medium. For example, DG44 cells with DHFR (dihydrofolate reductase deficient cells) defects cannot grow in a medium without hypoxanthine thymine. After the cells are transfected with a vector that can express DHFR, the transfected cells can not only grow in a medium without hypoxanthine thymine, but also grow in a medium containing a certain amount of MTX (methotrexate).

Both expression vectors and cloning vectors typically contain one or more gene transcription promoters, which are either recognized by prokaryotic or eukaryotic transcription mechanisms. Promoters used for eukaryotic cell transcription include but are not limited to cytomegalovirus (CMV) promoters, retroviral promoters, and monkey virus 40 (SV40) early promoters.

The expression vector can use commercially available vectors such as but not limited to vectors that can be used for eukaryotic cell system expression, such as pIRES, pDR, pUC18, etc.. Those skilled in this field can choose appropriate expression vectors based on host cells.

Based on the enzyme digestion patterns of known empty expression vectors, those skilled in the art can use conventional methods to insert the coding sequence of the fusion protein of the present invention into suitable restriction sites through restriction enzyme cleavage and splicing, to prepare a recombinant expression vector of the present invention.

The present invention also provides host cells expressing the fusion protein of the present invention, which contain the coding sequence of the fusion protein of the present invention. The preferred host cells are eukaryotic cells, such as but not limited to CHO, COS cells, 293 cells, RSF cells, etc.. As a preferred method of the present invention, the cells are CHO cells, which can effectively express the fusion protein of the present invention and obtain fusion proteins with good binding activity and stability.

The present invention also provides a method for preparing the fusion protein according to the invention with recombinant DNA, the steps of which include:
1) Providing nucleic acid sequences encoding fusion proteins;
2) Inserting the nucleic acid sequence of 1) into a suitable expression vector to obtain a recombinant expression vector;
3) Transfecting the recombinant expression vector of 2) into suitable host cells;
4) Culturing transformed host cells under suitable conditions for expression;
5) Collecting the supernatant and purifying the fusion protein product.

The introduction of the coding sequence into host cells can be achieved using various known techniques in the field, such as but not limited to: calcium phosphate precipitation, protoplast fusion, liposome transfection, electroporation, microinjection, reverse recording, phage transduction, and alkali metal ion methods.

The culture and expression of host cells can be found in Olander RM Dev Biol Stand 1996; 86:338. Cells and residues in the suspension can be removed by centrifugation to collect clear liquid. It can be identified by polyacrylamide gel electrophoresis.

The recombinant protein prepared by the above methods can be purified into a substantially homogeneous substance, such as a single band on SDS-PAGE electrophoresis. For example, when the recombinant protein is expressed in secretion, the protein can be separated by a commercial ultrafiltration membrane, such as Millipore, Pellicon, and other products, and the expression supernatant is first concentrated. The concentrated solution can be further purified by gel chromatography or purified by ion exchange chromatography. For example, anion exchange chromatography (DEAE, etc.) or cation exchange chromatography. Gel matrix may be polyacrylamide, dextran, polyamide and other matrices commonly used for protein purification. Q- or SP- groups are ideal ion exchange groups. Finally, methods such as hydroxyapatite adsorption chromatography, metal chelation chromatography, hydrophobic interaction chromatography, and reverse phase high performance liquid chromatography (RP-HPLC) can also be used to further refine and purify the purified products mentioned above. All the purification steps mentioned above can utilize different combinations to achieve a substantially uniform protein purity.

The expressed fusion protein can be purified using affinity chromatography columns containing specific antibodies, receptors, or ligands against the fusion protein. Depending on the characteristics of the affinity column used, the fused peptides bound to the affinity column can be eluted using conventional methods such as high salt buffer, changing pH, etc. Optionally, the amino or carboxyl terminus of the fusion protein may also contain one or more peptide fragments as protein tags. Any suitable tag can be used for the present invention. For example, the tag may be FLAG, HA, HA1, c-Myc, 6-His or 8-His, etc. These tags can be used to purify the fusion protein.

### Peptide linker

The present invention provides a fusion protein that optionally contains a peptide linker. The size and complexity of peptide linker may affect the activity of proteins. Usually, a peptide linker should have sufficient length and flexibility to ensure that the two proteins connected have sufficient degrees of freedom in space to perform their functions. At the same time, the formation of α-helix or β-fold in the peptide linker should be avoided affecting the stability of the fusion protein.

The length of the peptide linker is generally 0-20 amino acids, preferably 0-10 amino acids.

### Pharmaceutical Composition

The present invention also provides a pharmaceutical composition comprising an effective amount (e.g., 0.000001 to 90wt%; preferably 0.1-50wt%; more preferably, 5-40wt%) of the fusion protein of the present invention, and a pharmaceutically acceptable carrier.

Typically, the fusion protein of the present invention can be formulated in a non-toxic, inert, and pharmaceutically acceptable aqueous carrier medium, wherein the pH is usually about 5-8, preferably, about 6-8.

As used herein, the term "effective amount" or "effective dose" refers to the amount that can produce function or activity on humans and/or animals and is acceptable to humans and/or animals.

As used herein, "pharmaceutically acceptable" ingredients are those that are suitable for use in humans and/or mammals without excessive side effects (such as toxicity, irritation, and allergy), that is, substances with a reasonable benefit/risk ratio. The term "pharmaceutically acceptable carrier" refers to the carrier for administering the therapeutic agents, including various excipients and diluents.

The pharmaceutical composition of the present invention comprises a safe and effective amount of the fusion protein of the present invention and a pharmaceutically acceptable carrier. Such carriers include, but are not limited to saline, buffers, glucose, water, glycerol, ethanol, and a combination thereof. Typically the pharmaceutical preparation should match the mode of administration, and the pharmaceutical composition of the present invention may be made in injectable form, for example by conventional methods, with normal saline or aqueous solutions containing glucose and other adjuvants. The pharmaceutical composition should be manufactured under sterile conditions. The administration amount of an active ingredient is a therapeutically effective amount. The pharmaceutical preparation of the present invention can also be made into sustained-release preparations.

The effective amount of the fusion protein of the present invention may vary depending on the mode of administration and the severity of the disease to be treated. The choice of the preferred effective amount may be determined by one of ordinary skill in the art based on various factors (e.g., through clinical trials). The factors include, but are not limited to the pharmacokinetic parameters of the fusion protein, such as bioavailability, metabolism, half-life, etc; the severity of the disease to be treated of the patient, the patient's weight, the patient's immune status, and the route of administration. Usually, when the fusion protein of the present invention is administered daily at a dose of approximately 0.00001mg-50mg/kg animal body weight (preferably 0.0001mg-10mg/kg animal body weight), satisfactory results can be achieved. For example, due to the urgent requirements of treatment conditions, separate doses can be given several times a day, or the doses can be proportionally reduced.

### The main advantages of the present invention include:

(1) The fusion protein of the present invention has the advantages of accurate identification, synergistic effect and controllable toxicity.
(2) The fusion protein of the present invention can efficiently recognize key molecules in the immune response, such as TNFa, BAFF or APRIL.
(3) The fusion protein of the present invention can simultaneously bind to TNFa and BAFF, or APRIL, while inhibiting the biological functions of TNFa and BAFF, or APRIL.

The present invention is further illustrated below in conjunction with specific examples. It should be understood that the examples are not intended to limit the scope of the invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Primer

The primers involved in the present invention are shown in Table 1 below:

**Table 1 Primer Sequences**

| Primer | Sequence (5'-3') | SEQ ID NO: |
|---|---|---|
| KDP068 | CTTTGGCAAAGAATTGGG | 1 |
| KDP422 | GTCGCCAGTGCTCCCTTCAG | 2 |
| KDP410 | CTGAAGGGAGCACTGGCGACGGGCAGTGCTCCCAAAA TG | 3 |
| KDP411 | TATCACAGCTCTTGGGCTCCGCATTCGTTCCTTTCACTG | 4 |
| KDP134 | GAGCCCAAGAGCTGTGATA | 5 |
| BGHR | AACTAGAAGGCACAGTCGAGGC | 6 |
| KDP066 | CGAACATCGATTGAATTCC | 7 |
| KDP093 | TCTAGCATTTAGGTGACAC | 8 |
| KDP408 | CTGAAGGGAGCACTGGCGACGACGCGCCAGCCCCCAC GC | 9 |
| KDP409 | TATCACAGCTCTTGGGCTCGGCCGGTTTCGGCCGCGG | 10 |
| KDP412 | CTGAAGGGAGCACTGGCGACTCACTCAGCTGCCGCAAG GAG | 11 |
| KDP413 | TATCACAGCTCTTGGGCTCCCTGAGCTTGTTCTCACAGA AG | 12 |

### Example 1: Construction of TNFR2-BCMA-Fc fusion protein expression plasmid

The ribonucleotide sequence encoding the human TNFR2 amino acid sequence (Met1-Asp257) and the human IgG Fc amino acid sequence were synthesized by GenScript (USA), Inc. The ribonucleotide sequence encoding the human BCMA amino acid sequence (Gly6-Ala54) was synthesized by Genewiz (China) Inc.

The recombinant genes encoding the TNFR2-BCMA-Fc fusion protein were linked using a two-step polymerase chain reaction (PCR) method. The first step is to amplify the human TNFR2 (Met1-Asp257) gene using PCR method (high fidelity polymerase Pfx, Invitrogen):
5' end primer KDP068 (SEQ ID NO: 1) : 5'-CTTTTGGCAAAGAATTGGG-3', located on the vector at the 5' end of the gene.
3' end primer KDP422 (SEQ ID NO: 2): 5'-GTCGCCAGTGCTCCCTTCAG-3', which is a specific primer for TNFR2 gene.

Similarly, the gene of BCMA (Gly6-Ala54) was amplified using PCR method:
5' end primer KDP410 (SEQ ID NO: 3):
   5'-CTGAAGGGAGCACTGGCGACGGGCAGTGCTCCCAAAATG-3';
3' end primer KDP411 (SEQ ID NO: 4):
   5' -TATCACAGCTCTTGGGCTCCGCATTCGTTCCTTTCACTG-3' ;

Similarly, the gene of Human IgG1 Fc (Glu99-Gly329) was amplified using PCR method:
5' end primer KDP134 (SEQ ID NO: 5):
   5'-GAGCCCAAGAGCTGTGATA-3';
3' end primer BGHR (SEQ ID NO: 6):
   5'-CTTTTGGCAAAGAATTGGG-3', located on the vector at the 3' end of the gene.

The first 20 nucleotide sequence of primer KDP410 is complementary to the nucleotide sequence of primer KDP422, while the first 19 nucleotide sequence of primer KDP411 is complementary to the nucleotide sequence of primer KDP134. This allows for the connection of these three PCR fragments during the overlap extension PCR process in the second step.

The above three PCR fragments were purified with DNA gel (Tiangen Biochemical Technology Co., Ltd., Beijing) and subjected to the second step of overlapping PCR.
5' end primer KDP066 (SEQ ID NO: 7), 5'- CGAACATCGATTGAATTCC-3'.
3' end primer KDP093 (SEQ ID NO: 8), 5'- TCTAGCATTTAGGTGACAC-3'.

There is a Not I restriction site before the transcription start site of TNFR2 gene and a Xba I restriction site at the 3' end of IgG1 Fc stop codon. After gel purification of the DNA fragments obtained through overlap extension PCR, Not I/Xba I double restriction enzyme digestion (Takara) was performed. Then, the digested PCR fragments were cloned into mammalian cell expression vectors with the same enzyme digestion. This mammalian cell expression vector is an improved pcDNA3.1 (Invitrogen), where the neomycin resistant gene in pcDNA3.1 was replaced by the DHFR (dihydrofolate reductase) gene. The improved vector is suitable for screening mammalian cells with high expression of stable transfected proteins. The recombinant plasmid was transfected into DH5a competent bacteria. The positive colonies containing the correct recombinant plasmid were identified by colony PCR, and the recombinant plasmid was purified.

The amino acid sequence of the TNFR2-BCMA-Fc fusion protein (SEQ ID NO.: 19) is shown in Figure 1C, and the specific amino acids are as follows:
Fragment 1. positions 1-235 of TNFR2 amino acid sequence (SEQ ID NO.: 13) ;
Fragment 2. positions 6-54 of the BCMA amino acid sequence (SEQ ID NO.:16);
Fragment 3. positions 99-329 of the human IgG1 amino acid sequence (accession number UniProtKB-P01857) (SEQ ID NO.: 17).

### Example 2: Construction of TNFR2-BAFFR-Fc fusion protein expression plasmid

The TNFR2-BAFFR-Fc fusion protein gene was constructed by the method of Example 1. The ribonucleotide sequence encoding the amino acid sequence of human BAFFR (Asp12-Ala46) was synthesized by Genewiz (China) Inc.

In the first step, the human TNFR2 (Met1-Asp257) gene and the human IgG1 Fc (Glu99-Gly329) gene were amplified by PCR (high-fidelity polymerase Pfx, Invitrogen) (same method as Example 1).

Similarly, the gene of BCMA (Asp12-Ala46) was amplified using PCR method:
5' end primer KDP408 (SEQ ID NO: 9):
   5' -CTGAAGGGAGCACTGGCGACGACGCGCCAGCCCCCACGC-3';
3' end primer KDP409 (SEQ ID NO: 10):
   5'-TATCACAGCTCTTGGGCTCGGCCGGTTTCGGCCGCGG-3'.

The first 20 nucleotide sequence of primer KDP408 is complementary to the nucleotide sequence of primer KDP422, while the first 19 nucleotide sequence of primer KDP409 is complementary to the nucleotide sequence of primer KDP134. This allows for the connection of these three PCR fragments during the overlap extension PCR process in the second step.

After the above three PCR fragments were purified by DNA gel (Tiangen Biochemical Technology Co., LTD., Beijing), the second step of overlapping PCR (the same method as Example 1) was carried out. The above three PCR fragments were linked in the corresponding order to construct the coding gene of TNFR2-BAFFR-Fc fusion protein. The constructed recombinant gene was then cloned into the mammalian cell expression vector by the same method as in Example 1.

The amino acid sequence of TNFR2-BAFFR-Fc fusion protein (SEQ ID NO.: 20) is shown in Figure 1D, and the specific amino acids are as follows:
Fragment 1. positions 1-235 of TNFR2 amino acid sequence (SEQ ID NO.: 13);
Fragment 2. positions 12-46 of the BAFFR amino acid sequence (SEQ ID NO.:16);
Fragment 3. positions 99-329 of the human IgG1 amino acid sequence (accession number UniProtKB-P01857) (SEQ ID NO.: 17).

### Example 3: Construction of TNFR2-TACI-Fc fusion protein expression plasmid

The TNFR2-TACI-Fc fusion protein gene was constructed by the method of Example 1. The ribonucleotide sequence encoding the amino acid sequence of human TACI (Ser68-Arg109) was synthesized by Genewiz (China) Inc.

In the first step, the human TNFR2 (Met1-Asp257) gene and the human IgG1 Fc (Glu99-Gly329) gene were amplified by PCR (high-fidelity polymerase Pfx, Invitrogen) (same method as Example 1).

Similarly, the gene of TACI (Ser70-Arg109) was amplified using PCR method:
5' end primer KDP412 (SEQ ID NO: 11):
   5'-CTGAAGGGAGCACTGGCGACTCACTCAGCTGCCGCAAGGAG-3';
3' end primer KDP413 (SEQ ID NO: 12):
   5'-TATCACAGCTCTTGGGCTCCCTGAGCTTGTTCTCACAGAAG-3'.

The first 20 nucleotide sequence of primer KDP412 is complementary to the nucleotide sequence of primer KDP422, while the first 19 nucleotide sequence of primer KDP412 is complementary to the nucleotide sequences of primer KDP134. This allows for the connection of these three PCR fragments during the overlap extension PCR process in the second step.

After the above three PCR fragments were purified by DNA gel (Tiangen Biochemical Technology Co., LTD., Beijing), the second step of overlapping PCR (the same method as Example 1) was carried out. The above three PCR fragments were linked in the corresponding order to construct the coding gene of TNFR2-TACI-Fc fusion protein. The constructed recombinant gene was then cloned into the mammalian cell expression vector by the same method as in Example 1.

The amino acid sequence of TNFR2-TACI-Fc fusion protein (SEQ ID NO.: 18) is shown in Figure 1E, and the specific amino acids are as follows:
Fragment 1. positions 1-235 of TNFR2 amino acid sequence (SEQ ID NO.: 13);
Fragment 2. positions 68-109 of the TACI amino acid sequence (SEQ ID NO.:16);
Fragment 3. positions 99-329 of the human IgG1 amino acid sequence (accession number UniProtKB-P01857) (SEQ ID NO.: 17).

### Example 4: Establishing a Stable Expression Cell Line of Fusion Protein

The host cell used for stable expression of these fusion proteins is Chinese hamster ovary cell CHO-KS. CHO-KS cells were acclimated to suspension growth in FBS-free OptiCHO medium (Invitrogen) by gradually decreasing the FBS content of the medium for CHO-K1 cells growing in fetal bovine serum (FBS) medium until they were cultured in FBS-free medium. The neomycin resistant gene in the pcDNA3.1 vector containing the fusion protein gene was replaced by the rat glutamine synthase gene. The fusion protein expression plasmid was transfected into CHO-KS cells using the electrotransfection (Bio Rad, Gene Pulser Xcell) method. The transfected cells were cultured for 24-48 hours and then screened and cultured on a 96 well culture plate using limited dilution method. The screening medium was OptiCHO, 5 µg/ml recombinant human insulin and 10 µM aminosulfoxide methionine (MSX). Cells were cultured at 37 °C in an 8% CO₂ incubator. Three weeks later, ELISA method (alkaline phosphatase coupled sheep-anti-human IgG Fc antibody, Jackson ImmunoResearch Lab) was used to analyze the cell culture medium of each well with a cell population. The cell population with positive fusion protein expression was further expanded, subjected to ELISA detection, and then expanded to obtain a stable cell line with fusion protein expression.

### Example 5:Preparation, purification, and identification of fusion proteins

The cell lines stably expressing each fusion protein obtained in Example 4 were cultured and expanded. The cell culture medium was centrifuged, the supernatant was collected, and the fusion protein was purified from the supernatant using a Protein-A affinity column.

### Results and analysis

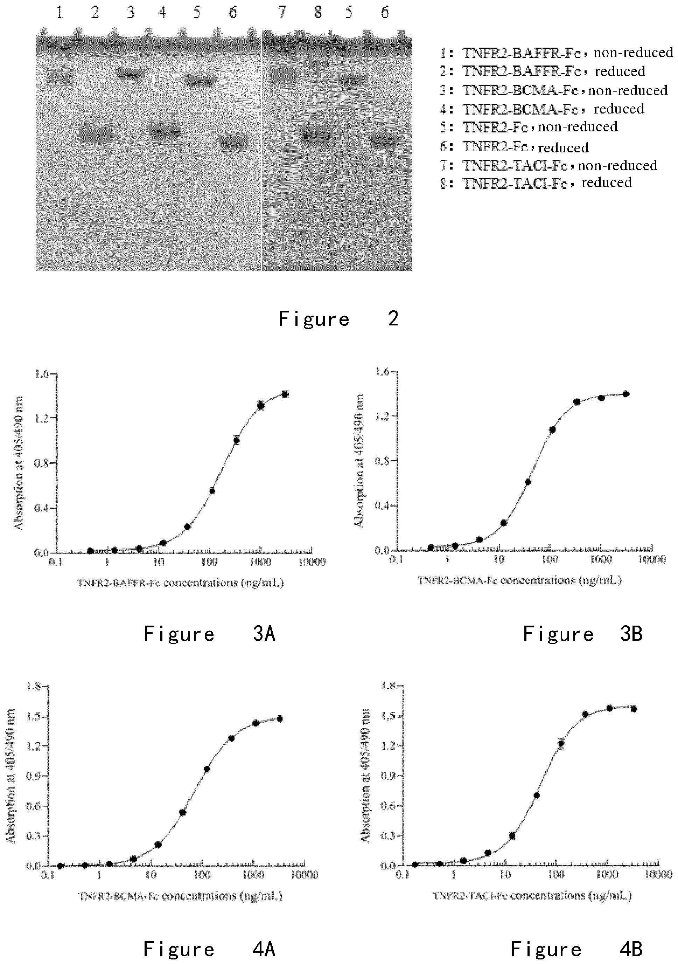

The fusion protein of the present invention is a homologous covalent dimer, and the molecular weights of TNFR2-BAFFR-Fc, TNFR2-BCMA-Fc, and TNFR2-TACI-Fc are not significantly different. The dimer is about 112 kDa (Figure 2, lanes 1, 3, 7, non-reducing SDS-PAGE), and the monomer is about 56 kDa (Figure 2, lanes 2, 4, 8, reducing SDS-PAGE), both of which are slightly larger than TNFR2-Fc (etanercept), of which the molecular weights of the dimer and monomer are 102 kDa and 51 kDa, respectively (Figure 2, lanes 5, 6).

### Example 6. Study of in vitro binding of TNFR2-BAFFR-Fc, TNFR2-BCMA-Fc and TNFR2-TACI-Fc with recombinant human TNFa

The *in vitro* binding activity of fusion proteins with recombinant human TNFa was studied by using ELISA method.

Recombinant human TNFa (Novoprotein) was dissolved in PBS (pH 7.4) to a final concentration of 0.8 µg/mL and added with 50 µL/well recombinant protein in a 96 well ELISA plate, and stored overnight in a refrigerator at 4 ° C. The next day, ELISA plates were washed three times with PBST (PBS containing 0.05% Tween-20), and 100 µL/well of blocking solution of PBST containing 3% BSA was added. ELISA plates were placed in an incubator at 37 °C for 1 hour. The fusion proteins were diluted in the binding solution of PBST containing 1% BSA to prepare three-fold serial dilutions. The blocking solution was removed and diluted proteins were added at 50 µL/well, respectively. The reaction was carried out for 1 hour at 37 °C. The solution was poured off, and the ELISA plate was washed three times with PBST. Then 50 µL/well of secondary antibody (alkaline phosphatase-conjugated sheep-anti-human IgG Fc antibody, Jackson ImmunoResearch Lab) was added and the reaction was performed in a 37 °C incubator for 1 hour. The chromogenic antibody was poured out, and 200 µL/well PBST cleaning solution was added to the ELISA plate. The ELISA plate was placed on a horizontal shaker for 5 minutes at 100 RPM, the cleaning solution was poured out, and the cleaning was repeated for 4 times. After being added with 50 µL/well antibody chromogenic solution (PNPP), the ELISA plate was placed in a 37 °C incubator for color development. An enzyme-linked immunosorbent assay was used to read the plate at a wavelength of 405 nm/490 nm.

### Results and analysis

Figure 3 shows that both fusion proteins, TNFR2-BAFFR-Fc and TNFR2-BCMA-Fc can specifically bind to human TNFα. TNFR2-BAFFR-Fc and TNFR2-BCMA-Fc bind to TNFa with an affinity EC₅₀ of 189 ng/mL and 46 ng/mL, respectively (FIGs. 3A and 3B).

### Example 7. Study of in vitro binding of TNFR2-BAFFR-Fc, TNFR2-BCMA-Fc and TNFR2-TACI-Fc with recombinant human BAFF

The binding activity of the fusion proteins to recombinant human BAFF was determined by ELISA.

Recombinant human BAFF was dissolved in 50 mM NaHCO3 (pH 9.6) to a final concentration of 1.0 µg/mL, and then 50 µL/well recombinant protein was added to a 96-well ELISA plate and stored in a refrigerator at 4°C overnight. The next day, ELISA plates were washed three times with PBST (PBS containing 0.05% Tween-20), and 100 µL/well of blocking solution of PBST containing 3% BSA was added. ELISA plates were placed in an incubator at 37 °C for 1 hour. The fusion proteins were diluted in the binding solution of PBST containing 1% BSA to prepare three-fold serial dilutions. The blocking solution was removed and diluted proteins were added at 50 µL/well, respectively. The reaction was carried out for 1 hour at 37°C. The solution was poured off, and the ELISA plate was washed three times with PBST. Then 50 µL/well of secondary antibody (alkaline phosphatase-conjugated sheep-anti-human IgG Fc antibody, Jackson ImmunoResearch Lab) was added and the reaction was performed in a 37 °C incubator for 1 hour. The chromogenic antibody was poured out, and 200 µL/ well PBST cleaning solution was added to the ELISA plate. The ELISA plate was placed on a horizontal shaker for 5 minutes at 100 RPM, the cleaning solution was poured out, and the cleaning was repeated for 4 times. After being added with 50 µL/well antibody chromogenic solution (PNPP), the ELISA plate was placed in a 37 °C incubator for color development. An enzyme-linked immunosorbent assay was used to read the plate at a wavelength of 405 nm/655 nm.

### Results and analysis

Figure 4 shows that both fusion proteins, TNFR2-BCMA-Fc and TNFR2-TACI-Fc can specifically bind to recombinant human BAFF *in vitro.* The binding affinity of TNFR2-BCMA-Fc and TNFR2-TACI-Fc fusion proteins to BAFF are 71 ng/mL and 50 ng/mL, respectively (Figures 4A and 4B).

### Example 8. Study of in vitro binding of TNFR2-BAFFR-Fc, TNFR2-BCMA-Fc and TNFR2-TACI-Fc with recombinant human APRIL

The binding activity of the fusion proteins to recombinant human APRIL was determined by ELISA.

Recombinant human BAFF was dissolved in 50 mM NaHCO₃ (pH 9.6) to a final concentration of 1.0 µg/mL, and then 50 µL/well recombinant protein was added to a 96-well ELISA plate and stored in a refrigerator at 4 °C overnight. The next day, ELISA plates were washed three times with PBST (PBS containing 0.05% Tween-20), and 100 µL/well of blocking solution of PBST containing 3%BSA was added. ELISA plates were placed in an incubator at 37 °C for 1 hour. The fusion proteins were diluted in the binding solution of PBST containing 1% BSA to prepare three-fold serial dilutions. The blocking solution was removed and diluted proteins were added at 50 µL/well, respectively. The reaction was carried out for 1 hour at 37 °C. The solution was poured off, and the ELISA plate was washed three times with PBST. Then 50 µL/well of secondary antibody (alkaline phosphatase-conjugated sheep-anti-human IgG Fc antibody, Jackson ImmunoResearch Lab) was added and the reaction was performed in a 37 °C incubator for 1 hour. The chromogenic antibody was poured out, and 200 µL/well PBST cleaning solution was added to the ELISA plate. The ELISA plate was placed on a horizontal shaker for 5 minutes at 100 RPM, the cleaning solution was poured out, and the cleaning was repeated for 4 times. After being added with50 µL/well antibody chromogenic solution (PNPP), the ELISA plate was placed in a 37 °C incubator for color development. An enzyme-linked immunosorbent assay was used to read the plate at a wavelength of 405 nm/655 nm.

### Results and analysis

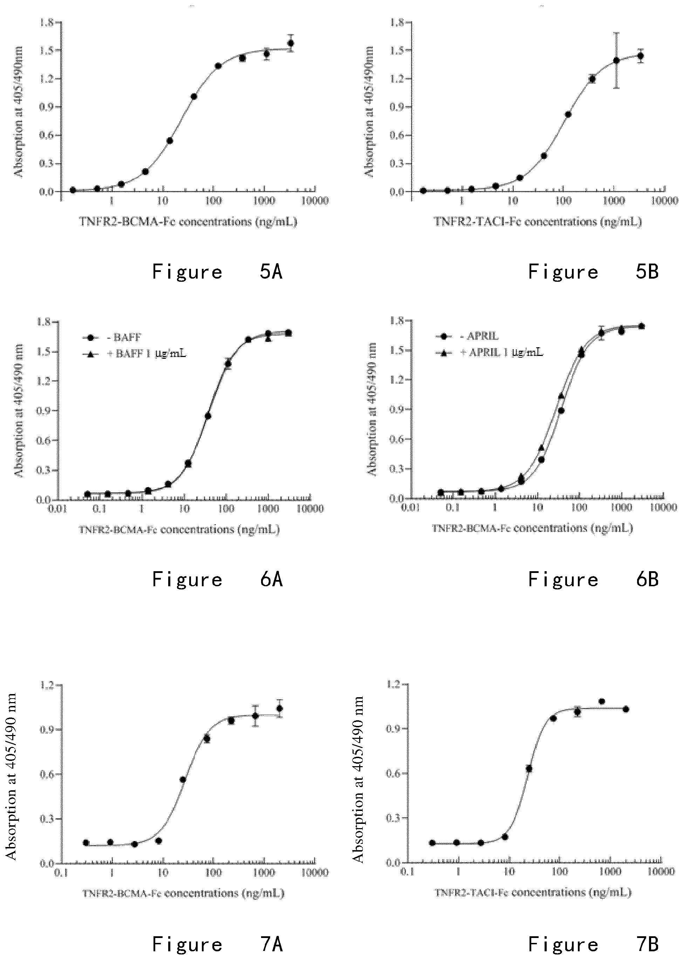

Figure 5 shows that both fusion proteins, TNFR2-BCMA-Fc and TNFR2-TACI-Fc can specifically bind to recombinant human APRIL *in vitro.* The binding affinity of TNFR2-BCMA-Fc and TNFR2-TACI-Fc to APRIL were 23 ng/mL and 104 ng/mL, respectively (Figures 5A and 5B).

### Example 9. TNFR2-BAFFR-Fc, TNFR2-BCMA-Fc and TNFR2-TACI-Fc fusion proteins simultaneously bind TNFa and BAFF or APRIL without affecting each other

The fusion proteins of the present invention bind TNFa and BAFF or APRIL with high affinity. Recombinant human TNFa (Novoprotein) was dissolved in PBS (pH 7.4) to a final concentration of 0.8 µg/mL, and rhTNFa was added at 50 µL/well to a 96-well ELISA plate and placed in a refrigerator at 4 ° C overnight. The next day, ELISA plates were washed three times with PBST (PBS containing 0.05% Tween-20), and 100 µL/well of blocking solution of PBST containing 3%BSA was added. ELISA plates were placed in an incubator at 37 °C for 1 hour. Fusion proteins were serially diluted 3-fold with binding solution (PBST containing 1% BSA) with or without 1 µg/mL BAFF or APRIL. The blocking solution was removed and diluted proteins were added to 50 µL/well, respectively. The reaction was carried out for 1 hour at 37 °C. The solution was poured off, and the ELISA plate was washed three times with PBST. Then 50 µL/well of secondary antibody (alkaline phosphatase-conjugated sheep-anti-human IgG Fc antibody, Jackson ImmunoResearch Lab) was added and the reaction was performed in a 37 °C incubator for 1 hour. The chromogenic antibody was poured out, and 200 µL/well PBST cleaning solution was added to the ELISA plate. The ELISA plate was placed on a horizontal shaker for 5 minutes at 100 RPM, the cleaning solution was poured out, and the cleaning was repeated for 4 times. After being added with 50 µL/well antibody chromogenic solution (PNPP), the ELISA plate was placed in a 37 °C incubator for color development. An enzyme-linked immunosorbent assay was used to read the plate at a wavelength of 405 nm/490 nm.

### Results and analysis

TNFR2-BCMA-Fc bind to TNFa in exactly the same way in solution with and without BAFF (Figure 6A), and there is no significant difference in the binding to TNFa in solution with and without APRIL (Figure 6B).

The experimental results demonstrate that BAFF or APRIL does not affect the binding of TNFR2-BCMA-Fc to TNFa, and TNFR2-BCMA-Fc can bind TNFa and BAFF or APRIL simultaneously without affecting each other.

### Example 10. Biological activity of TNFR2-BAFFR-Fc, TNFR2-BCMA-Fc and TNFR2-TACI-Fc fusion proteins in inhibiting TNFa-induced apoptosis

The biological activity of TNFR2 fusion protein was studied by neutralizing the biological activity of TNFa *in vitro.* TNFa biological activity was detected using mouse fibroblast L929 cytotoxicity assay. 5 ng/mL rhTNFa was mixed with serial dilutions of different concentrations of each fusion protein and then added to L929 cells cultured in 96-well plates. Actinomycin D (final concentration 20 µg/mL) was added to the medium. The viability of L929 cells was detected by crystalloid staining after 20 hours of incubation in a cell incubator.

### Results and analysis

Figure 7 shows the results of the study in which the fusion protein inhibited TNFa-induced apoptosis. The EC₅₀ of TNFR2-BCMA-Fc and TNFR2-TACI-Fc against TNFa-induced apoptosis in L929 cells was 27 ng/mL and 23 ng/mL, respectively (FIGs. 7A and 7B).

### Example 11. TNFR2-BAFFR-Fc, TNFR2-BCMA-Fc and TNFR2-TACI-Fc fusion proteins inhibit the growth of RPMI8226 cells stimulated by BAFF and APRIL in vitro

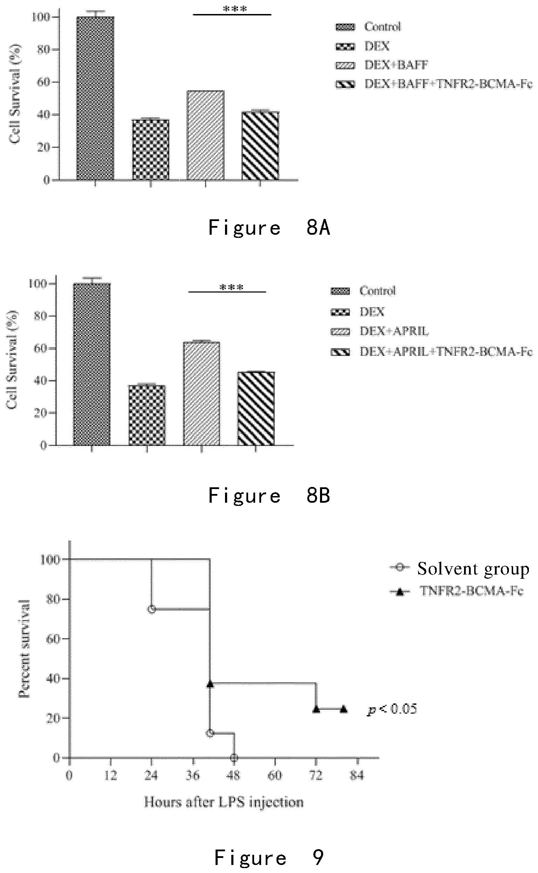

RPMI8226 is a human multiple myeloma cell line, and dexamethasone (DEX) can induce RPMI8226 cell death. BAFF or APRIL can inhibit the effect of DEX on inducing RPMI8226 cell death. We investigated the impact of the fusion proteins of the present invention on the aforementioned inhibitory effects of BAFF and APRIL. RPMI8226 cells were cultured in RPMI1660 medium (containing 10% FBS) on a 96 well plate, and various reagents as shown in Figure 8 were added. The cells were cultured in a cell incubator for 5 days, and then the number of live cells was detected using CCK-8.

### Results and analysis

Compared with the negative control cells (cultured in RPMI1660+10%FBS medium only), the survival rate of RPMI8226 cells in the presence of 0.1 µM DEX is 37%. 0.1 µg/mL BAFF inhibited DeX-induced cell death and increases the survival rate of RPMI8226 cells to 55%. 10 µg/mL of TNFR2-BCMA-Fc inhibits the function of BAFF, reduces the survival rate of BPMI8226 cells to 42%, showing significantly inhibitory effect on BAFF activity (p<0.0001) (Figure 8A). TNFR2-BCMA-Fc can also inhibit the function of APRIL of protecting RPMI8226 cells, reducing the survival rate of RPMI8226 cells from 63% to 46% (p<0.0001) (Figure 8B).

The results of this experiment demonstrate that the fusion proteins of the present invention has the function of inhibiting BAFF or APRIL of protecting the survival of RPMI8226 cells.

### Example 12. TNFR2-BAFFR-Fc, TNFR2-BCMA-Fc and TNFR2-TACI-Fc fusion proteins reduce the mortality of LPS-induced septic shock in mice

The effect of fusion proteins on LPS-induced septic shock death in mice was studied, and the biological activity of fusion proteins in neutralizing TNFa *in vivo* was evaluated. 16 male Balb/c mice aged 7-8 weeks were divided into 2 groups of 8 mice each. Each mouse was intraperitoneally injected with 1 mg of LPS, followed by intravenous injection of PBS and 6 mg/kg of TNFR2-BCMA-Fc into two groups of mice, respectively. The mouse status was observed for the following 80 hours, and the time of mouse death was recorded.

### Results and analysis

Figure 9 shows that within 24 h after LPS administration, two mice in the PBS group died, whereas no mice in the TNFR2-BCMA-Fc group died. Five more mice in the PBS group died within 41 hours, with a mortality rate of 88%, and all mice in the PBS group died within 48 hours. In the TNFR2-BCMA-Fc group, 5 mice died within 41 hours, with a mortality rate of 63%. One more mouse died within 72 hours, and a total of 6 mice died within 80 hours, with a mortality rate of 75%.

The results showed that TNFR2-BCMA-Fc reduces the mortality of LPS-induced shock in mice.

### Example 13. Reduction of the content of B lymphocytes in mouse by TNFR2-BAFFR-Fc, TNFR2-BCMA-Fc and TNFR2-TACI-Fc fusion proteins

The mice were injected with the fusion protein twice, and the content of B lymphocytes in the spleen of the mice was detected to evaluate the effect of the fusion protein on B lymphocytes.

Male C57BL/6 mice aged 6-7 weeks were randomly divided into groups based on body weight, with 5 mice in each group. Corresponding drugs were administered through the tail veins on D1 and D5. On D9, 100 µL of fundus blood was collected from each mouse and added to 100 µL of PBS-containing anticoagulant, 1 µL of anti-mouse B220(CD45R)-APC and anti-mouse CD3-FITC were added. After staining on ice for 30 minutes, 6 times the volume of flow hemolysin was added for lysis. After shaking, the cells were allowed to stand for 10 minutes, centrifuged at 800G for another 15 minutes, and resuspended in 200 µL of FACS working solution. Flow cytometry was used to detect the proportion of B220+ cells and CD3+ cells in the samples. All mice were sacrificed on D10, blood was collected, mice were dissected to take the spleen, and spleen lymphocytes were isolated. The proportion of B220+ and CD3+ cell populations in spleen lymphocytes were detected using the same method as above.

### Results and analysis

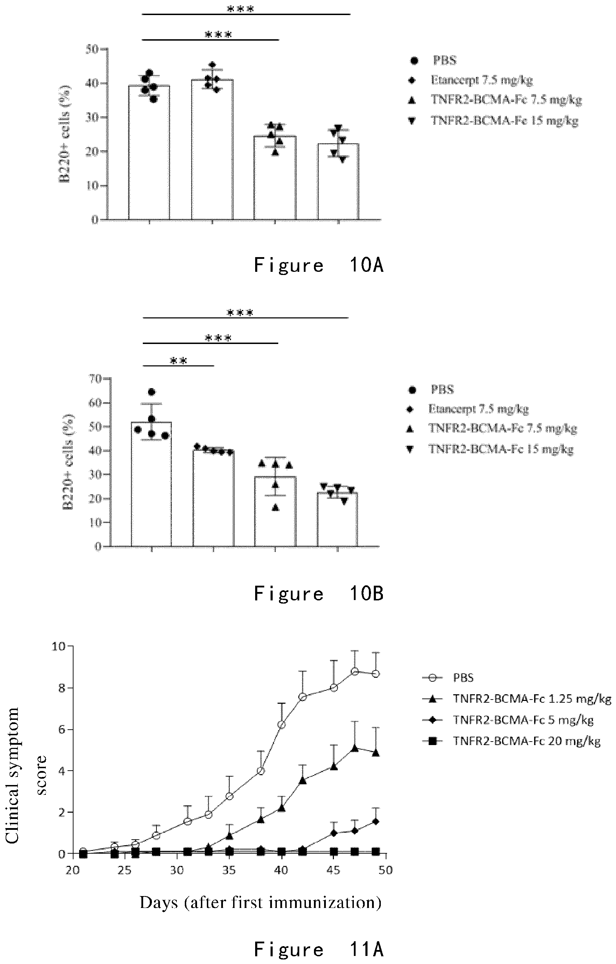

The membrane protein B220 is a biomarker of mouse B lymphocytes and is expressed by all B cells. Figure 10 shows that TNFR2-BCMA-Fc significantly reduces the content of B lymphocytes in the blood (Figure 10A) and spleen (Figure 10B) of mice. The doses of 7.5 mg/kg and 15 mg/kg of TNFR2-BCMA-Fc reduce the content of B lymphocytes in the blood by 37% and 43%, respectively, and reduce the content of B lymphocytes in the spleen by 44% and 56%, respectively. Etanercept does not have any effect on the amount of B lymphocytes in the blood, but it reduces the amount of B lymphocytes in the spleen (23% reduction), which is significantly weaker than the effect of the same dose of TNFR2-BCMA-Fc (44% reduction).

### Example 14. Effect of TNFR2-BCMA-Fc fusion protein on the inflammation of collagen induced arthritis (CIA) mouse model

The mouse model of CIA was established according to Schett et al., Arthritis Rheum. 52:1604-1611, 2005. DBA/1 mice (7 weeks old) were divided into 6 groups, with 9 mice in each group. Mice were immunized with collagen emulsion A (Bovine type II collagen + Complete Freund's Adjuvant) at D0. Mice were immunized with collagen emulsion B (Bovine type II collagen+ Incomplete Freund's Adjuvant) at D21. On D21, five groups of mice were intraperitoneally injected with three doses of TNFR2-BCMA-Fc (1.25, 5, and 20 mg/kg) and two doses of etanercept (Enbrel), respectively. The sixth group of mice were intraperitoneally injected with PBS. Additionally, one group consisting of 4 normal mice was set up. Administration were performed 3 times a week for a total of 4 weeks. The experiment was terminated at D49. The degree of inflammation in mice was assessed by clinical joint score and hindpaw thickness measurement. Clinical score was evaluated three times a week.

At the end point of the experiment, the entire left hind paw was fixed with 4% paraformaldehyde, and then soaked in 75% ethanol for preservation. HE staining and histopathological analysis were performed on the ankle and tarsophalangeal joint (Wuhan Seville Biotechnology Co., Ltd.). Pathological analysis was conducted using a semi quantitative method to evaluate the severity of synovial inflammation, pannus formation, bone erosion, and cartilage damage. After the experiment, the spleens of all the mice were taken, their weights were measured, and splenic lymphocytes were separated. Cells were stained with anti CD3 and anti B220 antibodies, and the number and content of T and B lymphocytes were measured using flow cytometry. The spleen weights and changes in B and T lymphocytes of each group of animals were compared. The titer of anti collagen antibodies in mouse serum at the end of the experiment was detected to evaluate the effect of TNFR2-BCMA-Fc on the production of anti collagen antibodies.

### Results and analysis

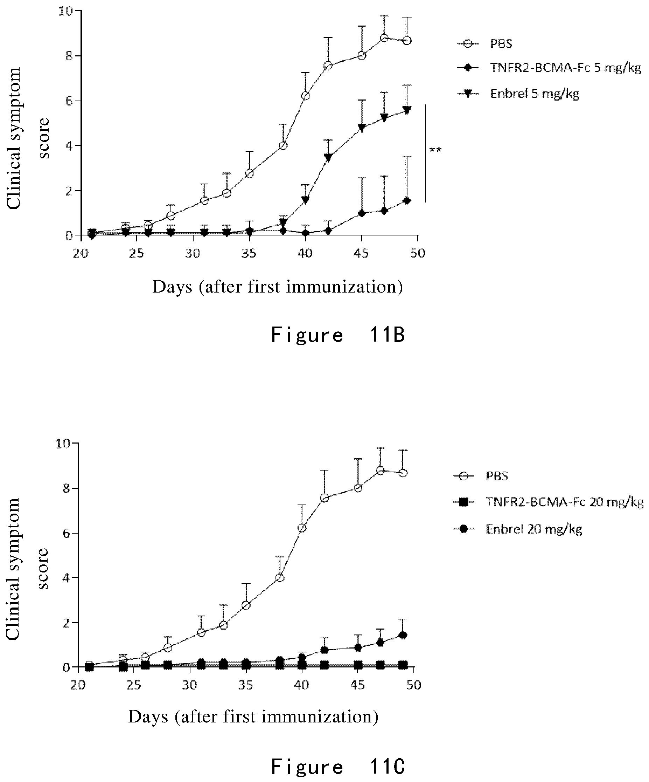

The research results show that compared with the control group, all three doses of TNFR2-BCMA-FC can inhibit the degree of inflammation in mice (evaluating the degree of paw swelling in mice), and the inhibitory effect was proportional to the dose (Figure 11A). Both doses of etanercept can also inhibit the degree of inflammation in mice, but compared to the same dose of TNFR2-BCMA-FC, the inhibitory effect is significantly lower than that of TNFR2-BCMA-FC (Figures 11B and 11C). Among them, the anti-inflammatory effect of 5 mg/kg etanercept is equivalent to that of 1.25 mg/kg TNFR2-BCMA-FC, significantly lower than that of 5 mg/kg TNFR2-BCMA-FC, with a statistically significant difference (p<0.01) (Figure 11B).

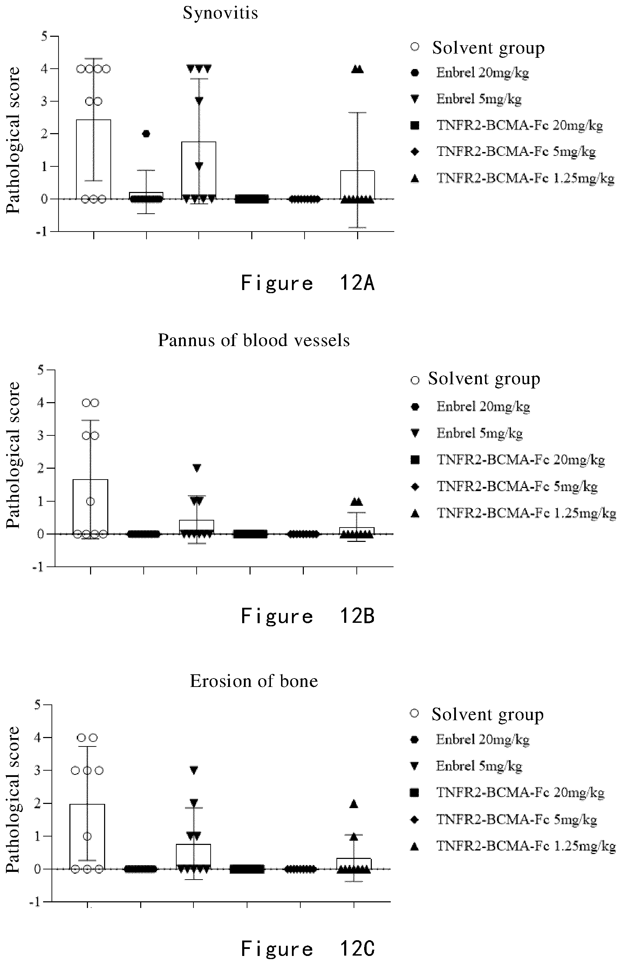

Pathological studies were conducted on the dorsal joint tissue of mice's toes (stained with hematoxylin-eosin), and the results show that all three doses of TNFR2-BCMA-FC can inhibit the occurrence of synovitis (Figure 12A), pannus (Figure 12B), and bone erosion (Figure 12C) in the joint tissue. The toe back tissues of all mice in the TNFR2-BCMA-FC 5 mg/kg and 20 mg/kg dose groups are the same as normal tissue. TNFR2-BCMA-FC at 1.25 mg/kg also partially protect tissues and is superior to Enbrel at 5 mg/kg. Similarly, the pathological research results of joint tissue also demonstrate that TNFR2-BCMA-FC has a significantly better inhibitory effect on inflammation in CIA mice compared to etanercept.

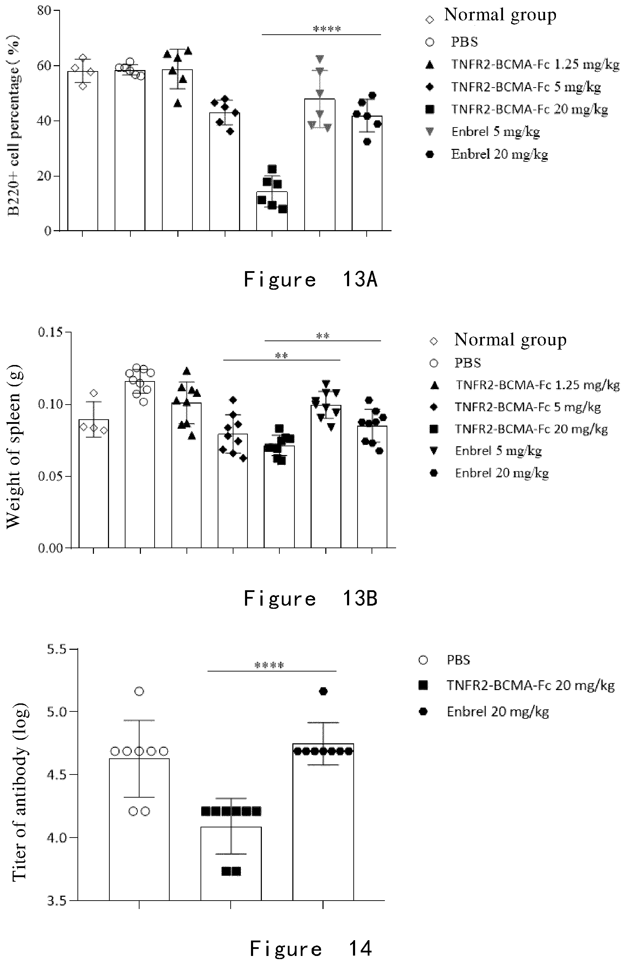

Based on the analysis of splenic B lymphocyte content in mice, TNFR2-BCMA-Fc at doses of 5 and 20 mg/kg is shown to reduce the percentage of B lymphocytes in the spleen (Figure 13A). The same dose of Enbrel also reduce the percentage of B lymphocytes in the spleen, but the effect is weaker than that of TNFR2-BCMA-Fc. Especially at the dose of 20 mg/kg, TNFR2-BCMA-Fc reduce the percentage of B lymphocytes significantly more than Enbrel (p<0.0001). Compared with normal mice, inflammation can cause splenomegaly (Figure 13B, PBS group). TNFR2-BCMA-Fc at doses of 5 and 20 mg/kg has the function of inhibiting splenomegaly (Figure 13B), and has a stronger inhibitory effect than Enbrel at the same dose.

TNFR2-BCMA-Fc can inhibit the titer of anti collagen antibodies (Figure 14). Compared with the PBS group, a dose of 20 mg/kg of TNFR2-BCMA-Fc significantly reduces the titer of anti collagen antibodies in the serum, while the same dose of Enbrel has no effect on antibody production.

All publications mentioned herein are incorporated by reference as if each individual document was cited as a reference in the present application. It should be understood that, after reading the above teachings of the present invention, those skilled in the art can make various modifications and changes. These equivalent forms are also within the scope defined by the claims appended hereto.

## Claims

1. A fusion protein, which comprises the the following fused elements:
(a) TNF receptor or active fragment thereof;
(b) APRIL/BAFF receptor or active fragment thereof, wherein the APRIL/BAFF receptor includes TACI, BCMA, BAFFR, and combinations thereof;
and optionally (c) antibody Fc region;
wherein, the fusion protein retains the biological activity of above elements (a) and (b).

2. The fusion protein of claim 1, wherein the fusion protein also has one or more of the following functions:
(a) activity of binding TNFα;
(b) inhibition of TNFα-induced inflammation;
(c) activity of binding BAFF or APRIL;
(d) inhibiting or blocking the BAFF/APPRIL pathway;
(e) reducing the number of B cells *in vivo*;
(f) reducing the content of B lymphocytes in the spleen or blood;
(g) inhibiting splenic enlargement;
(h) inhibition of APRIL/BAFF-induced diseases.

3. The fusion protein of claim 1, wherein the fusion protein has the following dimeric structure as shown in formula I or II:
X-Y-Z (I)
Y-X-Z (II);
wherein,
X is an extracellular segment of the TNF receptor;
Y is an extracellular segment of TACI, BCMA or BAFFR;
Z is none or an optional Fc region of human antibody;

4. The fusion protein of claim 1, wherein the amino acid sequence of the fusion protein is as shown in SEQ ID NO: 18 or 19 or 20.

5. The fusion protein of claim 3, wherein the X contains or has residues 1-235, 17-179, 17-140, 55-179, or 55-140 of the TNFR2 amino acid sequence (SEQ ID NO.:13); and/or
the Y contains or has:
(a) residues 1-109, 33-109, or 68-109 of the TACI amino acid sequence (SEQ ID NO.:14);
(b) residues 1-54, or 6-54 of the BCMA amino acid sequence (SEQ ID NO.:15); or
(c) residues 1-78, 12-78, or 12-46 of the BAFFR amino acid sequence (SEQ ID NO.:16); and/or
the Z contains or has residues 99-329 of the human IgG1 amino acid sequence (accession number UniProtKB-P01857).

6. A nucleic acid molecule encoding the fusion protein of claim 1.

7. A vector comprising the nucleic acid molecule of claim 6.

8. A genetically engineered cell comprising the vector of claim 7 or having the nucleic acid molecule of claim 6 integrated into its genome.

9. A method of producing the fusion protein of claim 1, which comprises the steps of:
culturing the genetically engineered cell of claim 8under conditions suitable for expression, thereby expressing the fusion protein; and
isolating or purifying the fusion protein.

10. A pharmaceutical composition, comprising the fusion protein of claim 1 and a pharmaceutically acceptable carrier thereof.

11. Use of the fusion protein of claim 1, the nucleic acid molecule of claim 6, the vector of claim 7, or the genetically engineered cell of claim 8, for the preparation of one or more compositions selected from the following group:
(a) composition for inhibiting BAFF/APPRIL signaling pathway;
(b) composition for treating immune diseases, preferably, the immune diseases are selected from the following group: rheumatoid arthritis (RA), ankylosing spondylitis (AS), psoriasis (PS), psoriatic arthritis (PsA), juvenile idiopathic arthritis (JIA), systemic lupus erythematosus (SLE), Behcet's disease (BD), multiple sclerosis (MS), Sjogren's syndrome (SS), Graves disease, Crohn's disease (CD), ulcerative colitis (UC), primary glomerulonephritis, IgA nephropathy, autoimmune vasculitis, polymyositis (PM), non-infectious uveitis, autoimmune hemolytic anemia (AIHA), autoimmune purpura (ATTP), N-methyl-d-aspartate receptor (NMDAR) encephalitis, myasthenia gravis, hydradenitis suppurativa (HS), myelin oligodendrocyte glycoprotein spectrum disorder (MOGSD), and neuromyelitis optica spectrum disorder (NMOSD), and combinations thereof; and/or
(c) composition for treating B cell proliferation-related diseases, preferably, the B cell proliferation related diseases include multiple myeloma, chronic lymphocytic leukemia, megaglobulinemia, and plasma cell leukemia.

12. A composition for (a) treating immune diseases; or (b) a method for treating B cell proliferation-related diseases, which comprises the step of administering the fusion protein of claim 1 to a subject in need thereof.
